# EUROPÄISCHE PATENTSCHRIFT

(11) **EP 0 727 204 B1**
(45) Veröffentlichungstag und Bekanntmachung des Hinweises auf die Patenterteilung: **09.07.1997**
(21) Anmeldenummer: 96100389.4
(22) Anmeldetag: 12.01.1996
(51) Int. Cl.: A61K 7/50, A61K 7/06

(54) **Haarwaschmittel**
Shampoo
Shampooing

(30) Priorität: 15.02.1995 DE 19504914
(43) Veröffentlichungstag der Anmeldung: 21.08.1996
(73) Patentinhaber: GOLDWELL GmbH, D-64297 Darmstadt (DE)
(72) Erfinder: Hinz, Sabine, D-64319 Pfungstadt (DE); Heinz, Dieter, D-65462 Gustavsburg (DE)

(56) Entgegenhaltungen:
- EP-A- 0 056 595
- EP-A- 0 342 054
- EP-A- 0 535 447
- FR-A- 2 385 793
- US-A- 4 374 125
- DATABASE WPI Week 7922 Derwent Publications Ltd., London, GB; AN 79-41525B & JP-A-54 050 513 , 20.April 1979

## Beschreibung

Die vorliegende Erfindung betrifft ein flüssiges Haarwaschmittel auf wäßriger Basis, das konditionierend auf die Haare wirkt, ihnen Glanz und Volumen sowie eine verbesserte Naß- und Trockenkämmbarkeit verleiht und insbesondere bei der Anwendung auf trockenem Haar dieses glänzend, weich und geschmeidig macht.

Ein solches erfindungsgemäßes Shampoo mit derart verbesserten Eigenschaften enthält mindestens ein anionisches, nichtionisches und/oder zwitterionisches Tensid und ein Gemisch aus mindestens 0,1 Gew.-% Milchsäure, mindestens 0,05 Gew.-% Citronensäure und mindestens 0,05 Gew.-% Pyrrolidoncarbonsäure (d.h., 5-Oxo-pyrrolidin-2-carbonsäure, auch unter der Bezeichnung Pyroglutaminsäure bekannt), jeweils berechnet auf die Gesamtzusammensetzung.

Der Zusatz dieses Gemisches zu an sich bekannten konventionellen Shampoos verleiht diesen die eingangs erwähnten Eigenschaften.

Aus der EP-A-0 056 595 sind bereits Haarbehandlungsmittel bekannt, die eine synergistische Kombination aus Betain und mindestens einer aliphatischen organischen Säure als haarkonditionierenden Bestandteil enthalten. Pyrrolidoncarbonsäure ist dort nicht erwähnt.

Die Wirkung der erfindungsgemäßen Haarwaschmittel kann noch verbessert werden durch den Zusatz weiterer organischer Säuren, ausgewählt aus der Gruppe Glykolsäure, Apfelsäure, Glyoxylsäure, Weinsäure und/oder Brenztraubensäure, vorzugsweise in einer Menge von mindestens 0,01, insbesondere mindestens 0,05 Gew.-%, berechnet auf die Gesamtzusammensetzung des Shampoos.

Die Obergrenze der organischen Säuren ist nicht kritisch und wird vor allem durch den gewünschten pH-Wert der erfindungsgemäßen Shampoos bestimmt. Sie liegt bei einem Gesamtanteil der genannten organischen Säuren von etwa 5 Gew.-%, vorzugsweise etwa 2,5 Gew.-%, berechnet auf die Gesamtzusammensetzung des Mittels.

Der Mindestanteil liegt bei etwa 0,20, insbesondere 0,5 Gew.-%. Diese organischen Säuren können als solche oder auch als Bestandteil von Pflanzen- und Obstsäften bzw. -extrakten zugesetzt werden.

Der bevorzugte pH-Wert liegt bei etwa 4 bis 7, insbesondere zwischen etwa 5 bis 5,5.

Ein weiterer bevorzugter Bestandteil der erfindungsgemäßen Shampoos ist Glycerin in einer Mindestmenge von etwa 0,5 Gew.-%, vorzugsweise etwa 1 bis etwa 10 Gew.-% des Mittels.

Bevorzugte Tenside im Rahmen der Erfindung sind anionische Tenside in einer Menge von mindestens 5 bis etwa 40 Gew.-% der Zusammensetzung.

Geeignete anionaktive Tenside sind solche vom Sulfat-, Sulfonat-, Carboxylat- und Alkylphosphat-Typ, insbesondere natürlich diejenigen, die in Shampoos üblicherweise zum Einsatz gelangen, beispielsweise die bekannten C₁₀-C₁₈-Alkylsulfate und insbesondere die entsprechenden Ethersulfate, beispielsweise C₁₂-C₁₄-Alkylethersulfat, Laurylethersulfat, insbesondere mit 1 bis 4 Ethylenoxidgruppen im Molekül, weiterhin Monoglyceridsulfate, Fettsäureamidsulfate, die durch Ethoxylierung und anschließende Sulfatierung von Fettsäurealkanolamiden erhalten werden, und deren Alkalisalze sowie Salze langkettiger Mono- und Dialkylphosphate, die milde, hautverträgliche Detergentien darstellen.

Im Rahmen der Erfindung weiterhin geeignete anionische Tenside sind α-Olefinsulfonate bzw. deren Salze und insbesondere Alkalisalze von Sulfobernsteinsäurehalbestern, beispielsweise das Dinatriumsalz des Monooctylsulfosuccinats und Alkalisalze langkettiger Monoalkylethoxysulfosuccinate.

Geeignete Tenside vom Carboxylat-Typ sind Alkylpolyethercarbonsäuren und deren Salze der Formel

R - (C₂H₄O)ₙ - O - CH₂ COOX,

worin R eine C₈-C₂₀-Alkylgruppe, vorzugsweise eine C₁₂-C₁₄-Alkylgruppe, n eine Zahl von 1 bis 20, vorzugsweise 2 bis 17, und X H oder vorzugsweise ein Kation der Gruppe Natrium, Kalium, Magnesium und Ammonium, das gegebenenfalls alkylhydroxyalkylsubstituiert sein kann, bedeuten, sowie Alkylamidopolyethercarbonsäuren der allgemeinen Formel

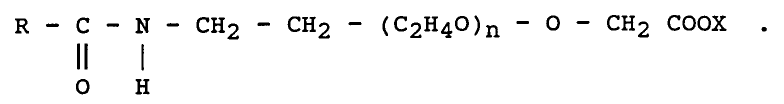

Derartige Produkte sind seit längerem bekannt und im Handel, beispielsweise unter den Handelsnamen "AKYPO" und "AKYPO-SOFT®".

Es ist besonders zweckmäßig, Mischungen aus mehreren anionischen Tensiden einzusetzen, beispielsweise ein Gemisch aus einem α-Olefinsulfonat und einem Sulfosuccinat, vorzugsweise im Verhältnis von 1 : 3 bis 3 : 1.

Im Gemisch mit anderen anionischen Tensiden ebenfalls einsetzbar sind Eiweiß-Fettsäure-Kondensationsprodukte an sich bekannter Struktur, insbesondere in Mengen zwischen etwa 0,5 und 5, vorzugsweise 1 bis 3 Gew.-% der Gesamtzusammensetzung des flüssigen Haarwaschmittels.

Eine Übersicht über die in flüssigen Körperreinigungsmitteln zum Einsatz gelangenden anionaktiven Tenside findet sich im übrigen in der Monographie von K.Schrader, "Grundlagen und Rezepturen der Kosmetika", 2. Aufl. (1989, Hüthig Buchverlag), S. 683 bis 691.

Der bevorzugte Mengenbereich an anionischen Tensiden in den erfindungsgemäßen flüssigen Körperreinigungsmitteln liegt zwischen etwa 5 und etwa 35 Gew.-%, insbesondere bei etwa 7,5 bis etwa 25 Gew.-%, besonders bevorzugt bei etwa 10 bis etwa 20 Gew.-%, berechnet auf die Gesamtzusammensetzung des Mittels.

Nichtionische Tenside gelangen im Rahmen der Erfindung vorzugsweise im Gemisch mit anionaktiven Tensiden zum Einsatz.

Ein bevorzugtes nichtionisches Tensid gehört dabei zu der Klasse der Alkylpolyglucoside der allgemeinen Formel

R - O - (R¹O)ₙ - Zₓ ,

worin R eine Alkylgruppe mit 8 bis 18 Kohlenstoffatomen, R¹ eine Ethylen- oder Propylengruppe, Z einen Saccharidrest mit 5 bis 6 Kohlenstoffatomen, n eine Zahl von 0 bis 10 und x eine Zahl zwischen 1 und 2,5 bedeuten.

Diese Alkylpolyglucoside sind in letzter Zeit insbesondere als ausgezeichnete hautverträgliche schaumverbessernde Mittel in flüssigen Wasch- und Körperreinigungsmitteln bekannt geworden.

Weitere nichtionische Tensidbestandteile sind beispielsweise langkettige Fettsäuremono- und -dialkanolamide, beispielsweise Cocosfettsäuremonoethanolamid und Myristinfettsäuremonoethanolamid, die auch als Schaumverstärker eingesetzt werden können.

Andere nichtionische Tenside sind beispielsweise die verschiedenen Sorbitanester, wie Polyethylenglykolsorbitanstearinsäureester, Fettsäurepolyglykolester oder auch Mischkondensate aus Ethylenoxid und Propylenoxid, wie sie beispielsweise unter der Handelsbezeichnung "Pluronics" im Verkehr sind.

Gemische aus anionaktiven Tensiden und Alkylpolyglucosiden, den bevorzugten nichtionischen Tensiden im Rahmen der Erfindung sowie deren Verwendung in flüssigen Körperreinigungsmitteln sind an sich bereits bekannt, beispielsweise aus der EP-A 70 074. Die dort beschriebenen Alkylpolyglucoside sind prinzipiell auch im Rahmen der vorliegenden Erfindung geeignet; ebenso die aus der EP-A 358 216 bekannten Gemische aus Sulfosuccinaten und Alkylpolyglucosiden.

Weitere im Gemisch mit anionaktiven Tensiden einsetzbare Tenside sind Aminoxide in einer Menge von etwa 0,25 bis etwa 5, vorzugsweise etwa 0,5 bis etwa 3,5 Gew.-%, berechnet auf die Gesamtzusammensetzung des Mittels.

Solche Aminoxide gehören seit langem zum Stand der Technik, beispielsweise C₁₂-C₁₈-Alkyldimethylaminoxide wie Lauryldimethylaminoxid, C₁₂-C₁₈-Alkylamidopropyl- oder -ethylaminoxide, C₁₂-C₁₈-Alkyldi(hydroxyethyl)- oder -(hydroxypropyl)aminoxide, oder auch Aminoxide mit Ethylenoxid- und/oder Propylenoxidgruppen in der Alkylkette.

Solche Aminoxide sind beispielsweise unter den Bezeichnungen "Ammonyx®", "Aromox®" oder "Genaminox®" im Handel.

Die erfindungsgemäßen Zusammensetzungen enthalten als weiteren möglichen Tensid-Bestandteil amphotere bzw. zwitterionische Tenside, wiederum vorzugsweise im Gemisch mit anionischen Tensiden, in einer Menge von etwa 0,1 bis etwa 5, vorzugsweise von etwa 0,5 bis etwa 3 Gew.-%, bezogen auf die Gesamtzusammensetzung. Als solche sind inbesondere die verschiedenen bekannten Betaine wie Fettsäureamidoalkylbetaine und Sulfobetaine, beispielsweise Laurylhydroxysulfobetain, zu nennen; auch langkettige Alkylaminosäuren haben sich als geeignet erwiesen.

Im einzelnen können Betaine der Struktur 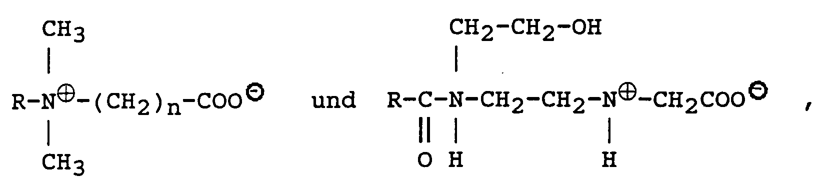 wobei R eine C₈-C₁₈-Alkylgruppe und n 1 bis 3 bedeuten, Sulfobetaine der Struktur 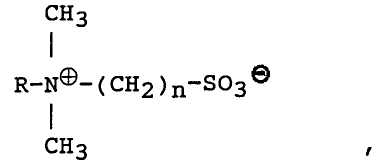 wobei R eine C₈-C₁₈-Alkylgruppe und n 1 bis 3 bedeuten, und Amidoalkylbetaine der Struktur 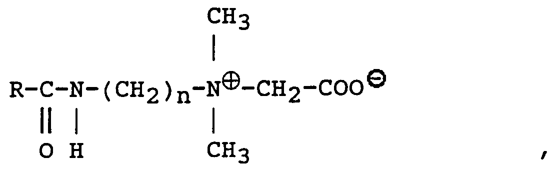 wobei R eine C₈-C₁₈-Alkylgruppe und n 1 bis 3 bedeuten, und langkettige Alkylaminocarbonsäuren verwendet werden.

Die erfindungsgemäßen Shampoos können selbstverständlich alle üblichen, in solchen Mitteln zum Einsatz gelangenden Stoffe enthalten.

Als solche seien beispielhaft Komplexbildner, Farbstoffe, Konservierungsmittel, pH-Regler, Viskositätsregler wie anorganische Salze, soweit sie nicht ohnehin in den Tensid-Ausgangsmischungen enthalten sind, Duftstoffe, Perlglanzmittel, Verdickungsmittel, Feuchthaltemittel, pflanzliche und tierische Öle wie Jojobaöl, etc. genannt.

Eine Auflistung solcher Zusatzstoffe findet sich ebenfalls bei Schrader, l.c., auf S.695 bis 722.

Besonders geeignete Zusatzstoffe für Shampoos sind haarkonditionierende Mittel. Als solche werden insbesondere kationische Polymere, vorzugsweise in einer Menge zwischen 0,1 bis 2, insbesondere 0,25 bis 1,25 Gew.-% der Gesamtzusammensetzung eingesetzt.

Aus der EP-A 337 354 ist die Verwendung von kationischen Polymeren mit Alkylpolyglucosid-Tensiden bereits bekannt; die dort auf S.3 bis S.7 aufgezählten kationischen Polymeren eignen sich auch als konditionierende Zusätze in den erfindungsgemäßen Zusammensetzungen.

Weitere konditionierende Zusätze sind die bekannten Eiweißhydrolysate, beispielsweise in einer Menge von 0,25 bis 5 Gew.-%, vorzugsweise 0,5 bis 2,5 Gew.-% der Gesamtzusammensetzung.

Weiterhin geeignet sind auch wasserlösliches Collagen bzw. wasserlösliche Collagen-Derivate.

Schließlich können auch, wie ebenfalls bereits bekannt, die verschiedenen Polysiloxane als konditionierende Mittel in den erfindungsgemäßen flüssigen Haarwaschmitteln mitverwendet werden. Deren bevorzugter Anteil liegt dabei etwa zwischen 0,5 und etwa 5, insbesondere 1 bis 3 Gew.-% der Gesamtzusammensetzung. Geeignet sind sowohl leichtflüchtige als auch schwerflüchtige cyclische oder lineare Polysiloxane, beispielsweise die unter dem Trivialnamen "Dimethicone" bzw. "Phenyldimethicone" sowie "Cyclomethicone" bekannten Silikonöle.

Geeignet sind beispielsweise auch die in der EP-A 398 177 beschriebenen Silikonderivate, die dort in Kombination mit Alkylpolyglucosiden in flüssigen Detergens-Zusammenetzungen eingesetzt werden.

Die erfindungsgemäßen Shampoos können auch Farbstoffe zur direkten oder oxidativen Färbung von Haaren enthalten, also sogenannte Tönungs- oder Färbeshampoos.

Die folgenden Beispiele dienen der Illustration der Erfindung. Die Herstellung der erfindungsgemäßen Produkte erfolgt durch Zusammenrühren der einzelnen Komponenten in Wasser, wobei auch Vormischungen verschiedener Bestandteile verwendet werden können.

### Beispiel 1

| Shampoo für normales Haar | |
|---|---|
| Natriumlaurylethersulfat | 9,00 (Gew.-%) |
| Dinatriumlaurylethersulfosuccinat | 3,00 |
| Decylglycosid (P.D.: 1,5) | 2,00 |
| PEG-55-propylenglykololeat | 2,00 |
| PEG-160-sorbitantristearat | 0,30 |
| PEG-3-distearat (Trübungsmittel) | 1,90 |
| Hydroxypropylguartrimoniumchlorid | 0,30 |
| Panthenol | 0,30 |
| Milchsäure | 0,10 |
| Citronensäure | 0,10 |
| Pyrrolidoncarbonsäure | 0,07 |
| Weinsäure | 0,05 |
| Glycerin | 1,00 |
| Klettenwurzelöl | 0,10 |
| Parfum | 0,60 |
| Konservierungsmittel | 0,35 |
| Natriumchlorid | 1,20 |
| Wasser | @ 100,00 |

Mit diesem Shampoo gewaschenes Haar zeigte im Vergleich zur Wäsche mit einem Shampoo identischer Zusammensetzung, das jedoch das Gemisch aus Milchsäure, Citronensäure, Pyrrolidoncarbonsäure und Weinsäure nicht enthielt, einen deutlich verbesserten Glanz, eine bessere Naß- und Trockenkämmbarkeit sowie ein erhöhtes Volumen.

### Beispiel 2

| Shampoo für dauergewelltes Haar | |
|---|---|
| Natriumlaurylethersulfat | 5,00 (Gew.-%) |
| Decylglycosid (P.D.: 1,5) | 2,50 |
| PEG-55-propylenglykololeat | 1,50 |
| Cocoamidopropylbetain | 1,50 |
| Laurylhydroxysultain | 0,80 |
| PEG-3-distearat (Trübungsmittel) | 1,90 |
| Quaternäres Cellulosederivat (Polymer JR 400) | 0,40 |
| Panthenol | 0,30 |
| Milchsäure | 0,10 |
| Citronensäure | 0,10 |
| Pyrrolidoncarbonsäure | 0,07 |
| Weinsäure | 0,05 |
| Glyoxylsäure | 0,03 |
| Apfelsäure | 0,03 |
| Brenztraubensäure | 0,05 |
| Glykolsäure | 0,05 |
| Glycerin | 1,00 |
| Mandelöl | 0,10 |
| Parfum | 0,60 |
| Weizenproteinhydrolysat | 0,30 |
| Konservierungsmittel | 0,35 |
| Natriumchlorid | 0,50 |
| Wasser | @ 100,00 |

Es wurde ein gegenüber dem Shampoo nach Beispiel 1 noch verbesserter Effekt erzielt.

### Beispiel 3

| Shampoo für gefärbtes Haar | |
|---|---|
| Natriumlaurylethersulfat | 5,00 (Gew.-%) |
| Cocoamidopropylbetain | 1,50 |
| Decylglycosid (P.D.: 1,5) | 2,00 |
| PEG-55-propylenglykololeat | 1,50 |
| Laurylhydroxysultain | 0,80 |
| PEG-3-distearat (Trübungsmittel) | 1,90 |
| Quaternäres Cellulosederivat (Polyquaternium-10) | 0,40 |
| Panthenol | 0,30 |
| Milchsäure | 0,10 |
| Citronensäure | 0,10 |
| Pyrrolidoncarbonsäure | 0,07 |
| Weinsäure | 0,05 |
| Glyoxylsäure | 0,03 |
| Apfelsäure | 0,03 |
| Brenztraubensäure | 0,05 |
| Glykolsäure | 0,05 |
| Glycerin | 1,00 |
| Triglycerin | 0,20 |
| Parfum | 0,60 |
| Aloe-Öl | 0,10 |
| Konservierungsmittel | 0,35 |
| Weizenproteinhydrolysat | 0,30 |
| Natriumchlorid | 0,50 |
| Wasser | @ 100,00 |

Die Wirkung des Produkts entsprach etwa derjenigen des Produkts nach Beispiel 2.

### Beispiel 4

| Shampoo für trockenes Haar | |
|---|---|
| Natriumlaurylethersulfat | 6,00 (Gew.-%) |
| Dinatriumlaurylethersulfosuccinat | 5,00 |
| Decylglycosid (P.D.: 1,5) | 5,00 |
| Laurylhydroxysultain | 0,90 |
| PEG-160-sorbitantristearat | 1,00 |
| PEG-3-distearat (Trübungsmittel) | 2,30 |
| Quaternäres Cellulosederivat (Polyquaternium-10) | 0,40 |
| Weizenkeimöl | 0,10 |
| Panthenol | 0,30 |
| Milchsäure | 0,10 |
| Citronensäure | 0,50 |
| Pyrrolidoncarbonsäure | 0,10 |
| Weinsäure | 0,10 |
| Apfelsäure | 0,10 |
| Brenztraubensäure | 0,10 |
| Glycerin | 1,50 |
| Triglycerin | 0,50 |
| Parfum | 0,60 |
| Weizenproteinhydrolysat | 1,00 |
| Konservierungsmittel | 0,35 |
| Natriumchlorid | 0,75 |
| Wasser | @ 100,00 |

Haarwäsche mit diesem Shampoo verlieh dem Haar feinen Glanz, Volumen und ausgezeichnete Naß- und Trockenkämmbarkeit im Vergleich zu einem konventionellen Shampoo.

### Beispiel 5

| Antischuppen-Shampoo | |
|---|---|
| Natriumlaurylethersulfat | 7,50 (Gew.-%) |
| Dinatriumlaurylethersulfosuccinat | 5,00 |
| Decylglycosid (P.D.: 1,5) | 2,00 |
| PEG-55-propylenglykololeat | 2,00 |
| PEG-3-distearat (Trübungsmittel) | 1,90 |
| Panthenol | 0,30 |
| Milchsäure | 0,10 |
| Citronensäure | 0,20 |
| Pyrrolidoncarbonsäure | 0,10 |
| Weinsäure | 0,10 |
| Glyoxylsäure | 0,05 |
| Brenztraubensäure | 0,10 |
| Apfelsäure | 0,03 |
| Brennesselöl | 0,10 |
| Glycerin | 1,00 |
| Parfum | 0,30 |
| Octopirox® (Antischuppenwirkstoff) | 0,40 |
| Konservierungsmittel | 0,35 |
| Allantoin | 0,10 |
| Natriumchlorid | 1,80 |
| Wasser | @ 100,00 |

### Beispiel 6

| Shampoo gegen fettiges Haar | |
|---|---|
| Natriumlaurylethersulfat | 9,00 (Gew.-%) |
| Decylglycosid (P.D.: 1,5) | 2,00 |
| PEG-3-distearat (Trübungsmittel) | 1,50 |
| Panthenol | 0,30 |
| Thymianöl | 0,10 |
| Milchsäure | 0,15 |
| Citronensäure | 0,40 |
| Pyrrolidoncarbonsäure | 0,10 |
| Weinsäure | 0,10 |
| Glykolsäure | 0,05 |
| Apfelsäure | 0,10 |
| Brenztraubensäure | 0,10 |
| Polyethylenglykol-6-isolaurylthioether in Cetylalkohol | 4,00 |
| Glycerin | 2,00 |
| Allantoin | 0,30 |
| Parfum | 0,60 |
| Konservierungsmittel | 0,35 |
| Natriumchlorid | 2,00 |
| Wasser | @ 100,00 |

## Patentansprüche

1. Haarwaschmittel, enthaltend mindestens ein anionisches, nichtionisches und/oder zwitterionisches (amphoteres) Tensid und ein Gemisch aus
a) mindestens 0,1 Gew.-% Milchsäure;
b) mindestens 0,05 Gew.-% Citronensäure; und
c) mindestens 0,05 Gew.-% Pyrrolidoncarbonsäure,
jeweils berechnet auf die Gesamtzusammensetzung des Mittels.

2. Haarpflegemittel nach Anspruch 1, dadurch gekennzeichnet, daß es zusätzlich mindestens 0,5 Gew.-% Glycerin enthält.

3. Haarpflegemittel nach Anspruch 1 oder 2, dadurch gekennzeichnet, daß es zusätzlich mindestens eine organische Säure, ausgewählt aus der Gruppe Glykolsäure, Apfelsäure, Glyoxylsäure, Weinsäure und/oder Brenztraubensäure, enthält.

4. Haarwaschmittel nach einem oder mehreren der Ansprüche 1 bis 3, dadurch gekennzeichnet, daß es 5 bis 50 Gew.-%, berechnet auf die Gesamtzusammensetzung, mindestens eines anionischen Tensids enthält.

## Claims

1. Hair shampoo containing at least one anionic, nonionic and (or) zwitterionic (amphoteric) surfactant and a mixture of
a) at least 0.1% by wt. lactic acid;
b) at least 0.05% by wt. citric acid; and
c) at least 0.05% by wt. pyrrolidone carboxylic acid,
all percentages calculated to the total composition.

2. Hair care composition according to claim 1, characterized in that it additionally contains at least 0.5% by wt. glycerol.

3. Hair care composition according to claim 1 or 2, characterized in that it additionally contains at least one organic acid selected from the group of glycolic acid, malic acid, glyoxylic acid, tartaric acid and (or) pyruvic acid.

4. Hair shampoo according to one or more of the claims 1 to 3, characterized in that it contains 5% to 50% by wt., calculated to the total composition, of at least one anionic surfactant.

## Revendications

1. Composition de shampooing, contenant au moins d'un agent tensio-actif anionique, nonionique et/ou zwitterionique (amphotére) et un mélange de
a) au moins 0,1% en poids d'acide lactique;
b) au moins 0,05% en poids d'acide citrique; et
c) au moins 0,05% en poids d'acide pyrrolidone carboxylique (acide pyroglutamique),
calculés par rapport à la composition totale.

2. Composition de shampooing selon la revendication 1, caractérisée en ce qu'elle contient en plus au moins 0,5% en poids de glycérine.

3. Composition de shampooing selon les revendications 1 au 2, caractérisée en ce qu'elle contient en plus au moins d'un acide organique, choisi du groupe d'acide glycolique, d'acide malique, d'acide glyoxylique, d'acide tartarique, et/ou d'acide pyruvique.

4. Composition de shampooing selon une on plusieurs des revendications 1 à 4, caractérisée en ce qu'elle contient entre 5 et 50% en poids par rapport à la composition totale au moins d'un agent tensio-actif anionique.
